# EUROPEAN PATENT APPLICATION

(11) **EP 4 242 661 A1**
(43) Date of publication of application: **13.09.2023**
(21) Application number: 21889583.7
(22) Date of filing: 04.11.2021
(51) Int. Cl.: G01N 33/68, G01N 33/573, C12Q 1/6883, A61K 38/17, A61K 38/43, A61P 19/00, A61P 19/10

(54) **COMPOSITION FOR REGULATING OSTEOCLAST DIFFERENTIATION, AND USE THEREOF**

(30) Priority: 05.11.2020 KR 20200146788
(71) Applicant: Industry-Academic Cooperation Foundation, Yonsei University, Seoul 03722 (KR)
(72) Inventor: LEE, Jin Woo, Seoul 06588 (KR); PARK, Kwang Hwan, Seoul 06509 (KR); LEE, Kyoung Mi, Goyang-si Gyeonggi-do 10423 (KR); JUNG, Sujin, Goyang-si Gyeonggi-do 10504 (KR)
(74) Representative: Thomann, William John
(86) International application number: PCT/KR2021/015902
(87) International publication number: WO 2022/098118

(57) **Abstract**

The present invention relates to a composition for regulating the differentiation of osteoclasts, and is very effective in preventing or treating bone-related diseases by effectively maintaining bone homeostasis by regulating bone metabolism, particularly the differentiation of bone marrow cells into osteoclasts.

## Description

### [Technical Field]

The present invention pertains to a composition for regulating osteoclast differentiation and uses thereof.

### [Background Art]

Osteoporosis, a typical bone-related disease, is a disease caused by an imbalance in the regulation of bone homeostasis between osteoclasts responsible for bone resorption and osteoblasts responsible for bone formation, and refers to a skeletal disorder characterized by weakened bone strength that can increase the risk of fracture (US National Institute of Health Definition NIH, 2000).

The imbalance in the regulation of bone homeostasis can occur due to causes such as aging, systemic disease, and menopause, and corresponds to a systemic disease in which bone density decreases and the microstructure of bone tissue deteriorates, thus increasing the risk of fractures. In other words, bone density is balanced and maintained through the disappearance of old bones and creation of new bones. However, due to aging, menopause and other causes, the replacement with new bones does not occur smoothly thus resulting in loosened bones, the repetition of which causes thinner bones with increased likelihood of cracking or breaking.

Osteoporosis is a disease in which the bone mass necessary for maintaining normal activities, such as a decrease in the calcium concentration of bone; that is, bone density is reduced and thus fractures are easily induced even upon light stimulation. The condition before progressing to osteoporosis is called osteopenia and refers to a state just before a hole in the bone, as the thickness of the bone continues to become thinner and lighter. In addition, osteomalacia refers to a condition in which bones are bent because calcium is not properly mixed with bones due to vitamin D deficiency or kidney diseases that excrete large amounts of calcium, resulting in soft bones. In addition, bone atrophy refers to reduction of bone degeneration, that is, the reduction of bone mass in already completed bone tissue.

Since the amount of bone in the human body is maintained through the regulation of bone homeostasis by the balance between osteoclasts and osteoblasts, it is important to develop treatments targeting molecules that play an important role in these cells. In other words, when the activity of osteoblasts that form bones decrease and the activity of osteoclasts that resorb bone increases, bone decomposition is promoted and diseases such as osteoporosis, in which bones become thinner and more easily broken, occur. Therefore, proteins capable of regulating the activity of osteoclasts and osteoblasts are being studied as therapeutic agents for bone diseases (Gregory R. Mundy, Journal of Bone and Mineral Metabolism (1996) 14:59-64; Chad Deal, Nature Clinical Practice RHEUMATOLOGY (2009) Vol 5 No 1; Kalervo Vaananen, Advanced Drug Delivery Reviews 57 (2005) 959-971). There is an urgent need to develop a therapeutic drug that can control the overall mechanism to maintain healthy bones, is effective even when taken for a long time, and has no side effects.

### [Disclosure of Invention]

### [Technical Problem]

One objective of the present invention is to provide a diagnostic composition for bone-related diseases.

Another objective of the present invention is to provide a diagnostic kit for bone-related diseases.

Still another objective of the present invention is to provide a method of providing information for diagnosing bone-related diseases.

Yet another objective of the present invention is to provide a device for diagnosing bone-related diseases,

Still yet another objective of the present invention is to provide a composition for regulating osteoclast differentiation.

A further objective of the present invention is to provide a composition for treating bone-related disease.

Another further objective of the present invention is to provide a method for treating bone-related disease.

Still another further objective of the present invention is to provide an agent for treating bone-related disease.

However, objects to be achieved by the present invention are not limited to the objects mentioned above, and other objects not mentioned herein will be clearly understood by those of ordinary skill in the art from the following description.

### [Technical Solution]

Hereinafter, various embodiments described herein are described with reference to the drawings. In the following description, numerous specific details, such as specific configurations, compositions and processes, etc., are provided for a thorough understanding of the present invention. However, certain embodiments of the present invention can be practiced without one or more of these specific details, or with other known methods and forms. In another embodiment, well-known processes and manufacturing techniques are not described in detail in order to not unnecessarily obscure the present invention. References throughout this specification to "one embodiment" or "an embodiment" means that a particular feature, configuration, composition, or characteristic described in connection with the embodiment is include in one or more embodiments of the present invention. Thus, the appearances of "in one embodiment" or "an embodiment" in various places throughout this specification do not necessarily refer to the same embodiment of the present invention. Additionally, the particular features, configurations, compositions, or properties may be combined in one or more embodiments in any suitable way.

All scientific and technical terms used herein without a specific definition within the specification have the same meaning as commonly understood by a person skilled in the art to which the present invention belongs.

One embodiment of the present invention is directed to a composition for diagnosing bone-related diseases.

In the present invention, the diagnostic composition may contain an agent for measuring the expression levels of the Pellino-1 (Protein Pellino Homolog 1) protein or of the gene encoding the same..

In the present invention, the agent for measuring the expression level of Pellino-1 protein may comprise at least one selected from the group consisting of antibodies, oligopeptides, ligands, PNAs (peptide nucleic acid), and aptamers, which bind specifically to the Pellino-1 protein, but is not limited thereto.

The "antibody" herein refers to protein molecules capable of specifically binding to the Pellino-1 protein, and the form of the antibody is not particularly limited. However, if it is a polyclonal antibody, a monoclonal antibody, or a substance with antigen-binding abilities, even parts of an antibody may be included, as well as all types of immunoglobulin antibodies. Additionally, special antibodies such as humanized antibodies can be included, and the antibody of the present invention includes not only complete forms with two full-length light chains and two full-length heavy chains, but also functional fragments of the antibody molecule. A functional fragment of an antibody molecule means a fragment with at least an antigen-binding function, and includes Fab, F(ab'), F(ab')2, and Fv but is not limited thereto.

The "oligopeptide" herein is a peptide consisting of 2 to 20 amino acids and may include a dipeptide, tripeptide, tetrapeptide and pentapeptide, but is not limited thereto.

The "PNA (Peptide Nucleic Acid)" herein refers to an artificially synthesized polymer similar to DNA and RNA, and was first introduced by Professors Nielsen, Egholm, Berg and Buchardt (at the University of Copenhagen, Denmark) in 1991. DNA has a phosphate-ribose backbone, whereas PNA has a backbone composed of repeating units of N-(2-aminoethyl)-glycine linked by peptide bonds. Thanks to this structure, PNA has a significantly increased binding affinity for DNA or RNA and a significantly increased stability, and thus is used in molecular biology, diagnostic analysis, and antisense therapy. PNA is disclosed in detail in Nielsen PE, Egholm M, Berg RH, Buchardt O (December 1991), "Sequence-selective recognition of DNA by strand displacement with a thymine-substituted polyamide", Science 254 (5037): 1497-1500.

The "aptamer" herein is a single strand of an oligonucleotide, and refers to a nucleic acid molecule with binding activity to the Pellino-1 protein. The aptamer can have various three-dimensional structures according to its base sequence and may have high affinity for a specific substance, such as an antigen-antibody reaction. A given aptamer can inhibit the activity of a given target molecule by binding to the given target molecule. The aptamer can be RNA, DNA, modified nucleic acid, or a mixture thereof, and may be linear or cyclic in shape.

The agent for measuring the expression level of the gene encoding the Pellino-1 protein may comprise at least one selected from the group consisting of primers, probes, and antisense nucleotides that specifically bind to the gene encoding the Pellino-1 protein, but is not limited thereto.

The "primer" herein is a fragment that recognizes a target gene sequence. It includes a pair of forward and reverse primers, but preferably provides analysis results with specificity and sensitivity. High specificity can be imparted when the primers amplify only the target gene sequence containing complementary primary binding sites while not causing non-specific amplification because the nucleic acid sequence of the primer is inconsistent with the non-target sequence present in the sample.

The "probe" herein refers to a substance which is capable of binding specifically to the target substance to be detected in a sample, and a substance that can specifically confirm the presence of a target substance in a sample through binding. Given probes are commonly used in the art, the type of probe is not limited but preferably can be PNA (peptide nucleic acid), LNA (locked nucleic acid), peptide, polypeptide, protein, RNA, or DNA, with PNA being the most preferred. More specifically, the probe is a biomaterial that includes those derived from or similar to those from living organisms or those manufactured in vitro. That may comprise enzymes, proteins, antibodies, microorganisms, animal and plant cells and organs, nerve cells, DNA, and RNA. DNA may comprise cDNA, genomic DNA and oligonucleotides, while RNA includes genomic RNA, mRNA, oligonucleotides, while proteins include antibodies, antigens, enzymes, peptides, and the like.

The "LNA (locked nucleic acids)" herein refers to a nucleic acid analog containing a 2'-O, 4'-C methylene bridge [J Weiler, J Hunziker and J Hall Gene Therapy (2006) 13, 496.502]. LNA nucleosides contain the common nucleic acid bases of DNA and RNA, and can form base pair according to the Watson-Crick base pairing rules. However, due to 'locking' of molecules attributable to the methylene bridge, the LNA is unable to form an ideal shape in the Watson-Crick bond. When the LNA is incorporated in the oligonucleotides of DNA or RNA, it can more rapidly pair with complementary nucleotide chains, thus increasing the stability of the double strand.

The "antisense" herein refers to oligomers with a sequence of nucleotide bases and an inter-subunit backbone, in which the antisense oligomer is hybridized with a target sequence in RNA by Watson-Crick base pair formation, allowing the formation of mRNA and RNA:oligomer heteroduplexes in the target sequence. Oligomers can have a sequence that has exactly or near complementarity to the target sequence.

Information regarding the Pellino-1 protein or the gene that encodes it is known, and thus those skilled in the art can readily design primers, probes or antisense nucleotides, which specifically bind to the gene that encodes the protein based on such information.

The diagnostic composition herein confirms increases or decreases in the expression levels of the Pellino-1 protein or the gene that encodes it among biological samples isolated from the individual of interest in comparison to the control group to diagnose bone-related diseases.

As used herein, the Pellino-1 protein refers to enzymes comprising Wing, FHA domain and RING-like domain, and is known to ubiquitinate IRAK1 and TBK1. Ubiquitination is one of the post-translational modification processes that regulate the activity of various proteins, and the ubiquitin conjugation cascade proceeds with the help of three enzymatic components: ubiquitin activating enzyme (E1), ubiquitin-conjugating enzyme (E2), and ubiquitin-protein ligase (E3). Pellino-1 normally promotes the ubiquitination of kinase receptor-interacting protein 1 (RIP1), IRAK, and NF-κB (nuclear factor-κB), and thus is known to promote signal complex formation in the Toll Like receptor (TLR) and Interleukins-1 receptor (IL-1R) signaling pathway. It also mediates the TRIF-dependent induction of type 1 interferons (IFNs). Pro-inflammatory cytokines induce osteoclast differentiation and inhibit osteoblast maturation. The amino acid sequence of Pellino-1 and its nucleic acid base sequence may be represented by SEQ ID NO:1 and SEQ ID NO:2, respectively, but is not limited thereto.

As used herein, the term "diagnosis" refers to confirming the presence of a pathological state or its characteristics. More specifically, it refers to determining bone-related diseases, particularly diseases caused by abnormal regulation of the differentiation of bone marrow cells into osteoclasts.

The bone-related diseases herein can result from the breakdown of coordinated activity between osteoblasts that generate bones in the body and osteoclasts that destroys bones in the body.

The osteoclast herein refers to large multinucleated cells that destroy or absorb unnecessary bone tissue during bone growth, and mature osteoclasts are multinucleated cells differentiated and formed from hematopoietic stem cells. Additionally, osteoblasts differentiated from mesenchymal stem cells survive for about 34 months and form new bones where activated osteoclasts decompose old bones. Numerous osteoblasts create a bone matrix, and bone formation is completed through the mineralization of the matrix. After bone formation is completed as such, about 70% of osteoblasts die while some survive by differentiating into osteocytes and bone lining cells. Bone-related diseases can occur when such homeostasis is continuously imbalanced.

With regard to the purpose of the present invention, the diagnostic composition for bone-related diseases herein targets Pellino-1 as a new target related to the differentiation of osteoclasts, unlike previously reported compositions, and is more effective in the diagnosis of bone-related diseases related to the regulation of osteoclast differentiation.

The bone-related diseases herein can be at least one of those selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis, but is not limited thereto.

The osteoporosis herein can be caused by factors at least one of those selected from the group consisting of primary osteoporosis due to aging, primary osteoporosis due to menopause, primary osteoporosis due to ovariectomy, glucocorticoid-induced osteoporosis, hyperthyroidism-induced osteoporosis, fixation-induced osteoporosis, heparin-induced osteoporosis, immunosuppression-induced osteoporosis, osteoporosis due to renal failure, inflammatory osteoporosis, osteoporosis due to Cushing's syndrome, rheumatoid osteoporosis, and osteoporosis caused by estrogen synthesis inhibitors, but is not limited thereto.

Another embodiment of the present invention is directed to a kit for diagnosing bone-related diseases that is comprised of the aforementioned composition.

The kit herein, using the composition of the present invention, can predict the presence of bone-related diseases when the expression level of the Pellino-1 protein or the gene that encodes it in the objective individual is increased or decreased in comparison to the normal control group. Preferably, when the expression level of the Pellino-1 protein or the gene that encodes it is higher or lower when compared to the normal control group, it can be predicted that there is an abnormality in the regulation of differentiation of bone marrow cells into osteoclasts.

The "control group" herein can be a normal control group, and more specifically may be obtained from a serum sample of a patient confirmed to have no bone-related diseases or may be an average to median value of the expression level of the Pellino-1 protein or the gene encoding it in a patent confirmed to have no bone-related disease after undergoing a bone density test, ultrasound and CT. The expression level of the marker protein or the gene encoding it in the control group can be compared to the expression level of the marker protein or the gene encoding it in a biological sample derived from patients of interest with bone-related diseases. Bone-related diseases can be diagnosed by determining whether the above expression levels have changed.

The kit herein, using the composition of the present invention, can predict that the objective individual has bone-related diseases caused by inhibition of osteoclast differentiation when the expression level of the Pellino-1 protein or the gene that encodes it in the objective individual is increased in comparison to the normal control group.

Additionally, when the expression level of the Pellino-1 protein or the gene that encodes it in the objective individual is decreased in comparison to the normal control group, it can be predicted that the objective individual has bone-related diseases caused by hyperdifferentiated osteoclasts.

In the diagnostic kit of the present invention, contents related to the Pellino-1 protein or the gene encoding it and bone-related diseases are the same as those described in the diagnostic composition, and thus the description thereof will be omitted in order to avoid excessive complexity of the present specification.

The kit herein may be a RT-PCR kit, a DNA ChIP kit, an ELISA kit, a protein ChIP kit, a rapid kit or a MRM (multiple reaction monitoring) kit, but is not limited thereto.

The kit of the present invention may further comprise one or more other component compositions, solutions, or devices suitable for the analysis method. For example, the kit of the present invention may further comprise essential elements necessary for carrying out the reverse transcription polymerase reaction. The reverse transcription polymerase reaction kit comprises a pair of primers specific to a gene encoding a marker protein. Each primer is a nucleotide with a sequence specific to the nucleic acid sequence of the given gene, and may have a length of about 7bp to 50bp, more preferably about 10bp to 30bp. In addition, the kit may comprise primers specific to the nucleic acid sequence of the control gene. In addition, the reverse transcription polymerase reaction kit may comprise test tubes or other suitable containers, reaction buffers (having various pH and magnesium concentrations), deoxynucleotides (dNTPs), enzymes such as Taq-polymerase and reverse transcriptase, DNase and RNase inhibitor DEPC-water, sterilized water, and the like.

In addition, the diagnostic kit of the present invention may comprise essential elements necessary for carrying out DNA ChIP. The DNA ChIP kit may comprise a substrate to which cDNA or oligonucleotides corresponding to genes or fragments thereof are attached, as well as reagents, agents, and enzymes for producing fluorescently labeled probes. Additionally, the substrate may comprise a cDNA or oligonucleotide corresponding to a control gene or a fragment thereof.

In addition, the diagnostic kit of the present invention may comprise essential elements necessary for carrying out ELISA. The ELISA kit may comprise antibodies specific to the protein. The antibody is a monoclonal antibody, polyclonal antibody, or recombinant antibody that has high specificity and affinity for a marker protein and little cross-reactivity to other proteins. The ELISA kit may also comprise antibodies specific to a control protein. Other ELISA kits may comprise reagents capable of detecting bound antibodies such as labeled secondary antibodies, chromophores, enzymes (e.g. conjugated with antibodies), substrates thereof or those capable of binding the antibody, and the like.

The fixture for the antigen-antibody binding reaction in the diagnostic kit of the present invention may be nitrocellulose film, PVDF film, well plate made of polyvinyl resin or polystyrene resin, slide glass made of glass, or the like, but is not limited thereto.

Additionally, the marker of the secondary antibody in the diagnostic kit of the present invention is preferably a conventional color developing agent that undergoes a color reaction. Fluorescein such as horseradish peroxidase, basic dephosphorylation enzyme (alkaline phosphatase), colloid gold, Poly L-lysine-fluorescein isothiocyanate (FITC), rhodamine-B-isothiocyanate (RITC), as well as dyes and the like may be used as markers, but is not limited thereto.

Additionally, the chromogenic substrate for inducing color development in the diagnostic kit of the present invention is preferably used according to a marker that undergoes color reactions, and may be TMB (3,3',5,5'-tetramethyl bezidine), ABTS [2,2'-azino-bis(3-ethylbenzothiazoline-6-sulfonic acid)], OPD (o-phenylenediamine), and the like . Here, it is more preferable that the chromogenic substrate is provided in a dissolved state in a buffer solution (0.1 M NaAc, pH 5.5). Chromogenic substrates such as TMB are degraded by HRPs used as a marker for the secondary antibody conjugate to generate chromogenic deposits, and the presence or absence of such marker proteins is detected by visually confirming the degree of deposition of the chromogenic deposits.

The washing solution in the kit for diagnosing anticancer drug resistance according to the present invention preferably comprises phosphate buffer, NaCl and Tween 20. More preferably, the washing solution is a buffer solution (PBST) consisting of 0.02 M phosphate buffer, 0.13 M NaCl, and 0.05% Tween 20. After the antigen-antibody binding reaction, the secondary antibody is allowed to react with the antigen-antibody complex, and then the resulting conjugate is washed 3 to 6 times with a suitable amount of the washing solution added to the fixture. As the reaction stop solution, a sulfuric acid (H₂SO₄) solution may be preferably used.

Still another embodiment of the present invention is directed to a method for providing information for diagnosing bone-related diseases.

The method of the present invention may comprise a step of measuring the expression level of Pellino-1 (Protein pellino homolog 1) or the gene encoding it in a biological sample isolated from the subject of interest.

The method of the present invention may be for screening for the presence of absence of bone-related diseases in a biological sample isolated from the subject of interest.

The "subject of interest" herein refers to mammals including humans, and may be one of those selected from the group consisting of humans, rats, mice, guinea pigs, hamsters, rabbits, monkeys, dogs, cats, cows, horses, pigs, sheep and goats, preferably human, but is not limited thereto.

The "human" herein may refer to a person who has had or is suspected of having bone-related diseases, and may refer to patients who need or is expected to receive appropriate treatment for bone-related diseases due to dysregulation of osteoclast differentiation, but is not limited thereto.

The "biological sample" herein refers to any material, biological fluid, tissue or cell obtained or derived from the subject with bone-related diseases or the subject suspected of having a bone-related disease and suspected of having dysregulation of osteoclast differentiation. It may comprise whole blood, leukocytes, peripheral blood mononuclear cells, buffy coat, plasma, and serum-containing blood, sputum, tears, mucus, nasal washes, nasal aspirate, breath, urine, semen, saliva, peritoneal washings, pelvic fluid, cystic fluid, meningeal fluid, amniotic fluid, glandular fluid, pancreatic fluid, lymph fluid, pleural fluid nipple aspirate, bronchial aspirate, synovial fluid, joint aspirate, organ secretions, cell, cell extract, or cerebrospinal fluid, but is not limited thereto.

In the present invention, the step for measuring the level of protein may carry out by protein ChIP analysis, immunoassay, ligand binding assay, MALDI-TOF (Matrix Assisted Laser Desorption/Ionization Time of Flight Mass Spectrometry) analysis, SELDI-TOF (Surface Enhanced Laser Desorption/Ionization Time of Flight Mass Spectrometry) analysis, radioimmunoassay, radioimmunodiffusion method, Oukteroni immunodiffusion method, rocket immunoelectrophoretic, tissue immunostaining, complement fixation assay, 2d electrophoresis assay, Liquid Chromatography-Mass Spectrometry (LC-MS), LC-MS/MS (Liquid Chromatography-Mass Spectrometry/Mass Spectrometry), Western blotting or ELISA (enzyme linked immunosorbent assay) using the composition of the present invention, but is not limited thereto.

In the present invention, the step for measuring the expression level of the gene encoding the protein may carry out by reverse transcription polymerase reaction (RT-PCR), competitive reverse transcription polymerase reaction (competitive RT-PCR), real-time reverse transcription polymerase reaction (Real-time; RT-PCR), RNase protection assay (RPA), Northern blotting, or DNA ChIP, but is not limited thereto.

In the methods for measuring the level of protein of the present invention, contents related to the Pellino-1 protein or the gene encoding it, agent for measuring expression levels, and bone-related diseases are the same as those described in the diagnostic composition, and thus are omitted to avoid excessive complexity.

In present invention, when the measured expression level of the Pellino-1 protein or the gene encoding the same in the biological sample is higher or lower than in a normal control, it may be predicted that the subject has a bone-related disease. More preferably, when the expression level of the Pellino-1 protein or the gene encoding the same is higher or lower than in a normal control, it may be predicted that the subject has a bone-related diseases due to dysregulation of osteoclast differentiation.

Additionally, when the expression level of the Pellino-1 protein or the gene encoding the same in the biological sample is higher than a normal control, it may be predicted that the subject has a bone-related disease due to inhibition of osteoclast differentiation.

Additionally, when the expression level of the Pellino-1 protein or the gene encoding the same in the biological sample is lower than the normal control group, it may be predicted that the subject has a bone-related disease due to hyperdifferentiation of osteoclasts.

The bone-related diseases herein can be at least one of those selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis, but is not limited thereto. Here, examples of bone-related diseases due to inhibition of osteoclast differentiation may comprise osteopetrosis, and examples of bone-related diseases due to hyperdifferentiation of osteoclasts may comprise osteoporosis, osteomalacia, osteopenia, bone atrophy fibrous dysplasia, Paget's disease, hypercalcemia, giant cell tumor, neoplastic destruction, cancer-related bone resorption disease, fracture, osteolysis, osteoarthritis, or rheumatoid arthritis, but is not limited thereto.

Yet another embodiment of the present invention is directed to a device for diagnosing bone-related diseases.

The measuring unit of the diagnostic device of the present invention can measure the expression level of a protein or gene using an agent that measures the expression level of a protein or the gene that encodes the same in a biological sample isolated from a subject of interest.

In the present invention, the subject of interest may be selected from the group consisting of humans, rats, mice, guinea pigs, hamsters, rabbits, monkeys, dogs, cats, cows, horses, pigs, sheep and goats, and preferably may be humans, but is not limited thereto.

In the present invention, the biological sample may be at least one selected from the group consisting of whole blood, leukocytes, peripheral blood mononuclear cells, buffy coat, plasma, and serum-containing blood, sputum, tears, mucus, nasal washes, nasal aspirate, breath, urine, semen, saliva, peritoneal washings, pelvic fluid, cystic fluid, meningeal fluid, amniotic fluid, glandular fluid, pancreatic fluid, lymph fluid, pleural fluid nipple aspirate, bronchial aspirate, synovial fluid, joint aspirate, organ secretions, cell, cell extract, or cerebrospinal fluid, but is not limited thereto.

In the present invention, the agent used in the measuring unit of the diagnostic device may be an agent that measures the expression level of the Pellino-1 (protein pellino homolog 1) or the gene that encodes it. More specifically, it may comprise at least one of those selected from the group consisting of antibodies, oligopeptides ligands, peptide nucleic acids (PNAs), and aptamers that specifically bind to the protein, and may comprise at least one of those selected from the group consisting of primers, probes, and antisense nucleotides that specifically bind to the gene.

In the present invention, the presence of absence of bone-related diseases, specifically bone-related diseases caused by abnormal regulation of the differentiation of bone marrow cells into osteoclasts, can be predicted by confirming the expression level of the protein or the gene encoding the same by using the aforementioned agent in the measuring unit of the diagnostic device.

In the present invention, the diagnostic device may further comprise a detection unit that predicts the presence or absence of bone-related diseases in the subject of interest using the protein or gene expression level obtained from the measuring unit.

In the present invention, the detection unit may diagnose bone-related diseases by generating and classifying information indicated that bone-related diseases due to inhibition of osteoclast differentiation have occurred or are likely to occur, when the expression level of the protein or gene obtained from the measuring unit is higher than the expression level of Pellion-1 protein or the gene in the control.

Additionally, in the present invention, the detection unit may diagnose bone-related diseases by generating and classifying information indicated that bone-related diseases due to hyperdifferentiation of osteoclasts have occurred or are likely to occur, when the expression level of the protein or gene obtained from the measuring unit is lower than the expression level of Pellion-1 protein or the gene in the control.

In the present invention, the bone-related diseases to be diagnosed by the diagnostic device may be at least one of those selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis, but is not limited thereto. Here, examples of bone-related diseases due to inhibition of osteoclast differentiation may comprise osteopetrosis, and examples of bone-related diseases due to hyperdifferentiation of osteoclasts may comprise osteoporosis, osteomalacia, osteopenia, bone atrophy fibrous dysplasia, Paget's disease, hypercalcemia, giant cell tumor, neoplastic destruction, cancer-related bone resorption disease, fracture, osteolysis, osteoarthritis, or rheumatoid arthritis, but is not limited thereto.

Still yet another embodiment of the present invention is directed to a composition for inhibiting osteoclast differentiation that inhibits differentiation of osteoclast.

In the present invention, the composition for inhibiting osteoclast differentiation may comprise at least one of those selected from the group consisting of the Pellino-1 protein, the gene that encodes the Pellino-1 protein, or the expression vector containing the gene encoding the Pellino-1 protein.

The Pellino-1 protein herein may be represented by SEQ ID NO:1, and may comprise other proteins with functionally equivalent characteristics.

In the pharmaceutical composition herein, proteins with functionally equivalent characteristics refer to proteins with at least 70% or more, preferably 80% or more, more preferably 90% or more sequence homology to the Pellino-1 protein as a result of the addition, substitution, or deletion of amino acids that have substantially equivalent physiological activity. As a non-limiting example, it may have 99% to less than 100%, 95% to less than 99%, 90% to less than 95%, 85% to less than 90%, or 80% to less than 85% sequence homology to the amino acid sequence of the Pellino-1 protein. It also may comprise, without limitation, those within a range that is obvious to those skilled in the art that the desired effect of the present invention is exhibited.

The gene encoding the Pellino-1 protein of the present invention may be included as such, preferably be included in a recombinant expression vector, but is not limited thereto.

The gene encoding the Pellino-1 protein of the present invention may be represented by SEQ ID NO:2, and may be provided in the form of a vector expressing the gene in cells for use in gene therapy or the like. The expression level of the gene can be increased by injecting an expression vector containing the gene encoding the Pellino-1 protein.

The recombinant expression vector herein is a recombinant expression vector capable of expressing a desired protein or peptide in a desired host cell, and refers to a gene construct containing essential regulatory elements operably linked to express the gene insert. The expression vector includes expression control elements such as start codons, stop codons, promoters, operators, and the like. The start codons and stop codons are generally considered to be part of a nucleotide sequence encoding a polypeptide, and when the gene construct is injected, it must be functional in the individual and must be in frame with the coding sequence. The promoter of the vector may be a constitutive or inducible promoter.

The recombinant expression vector herein may be one in which a promoter and a nucleotide sequence encoding the Pellino-1 protein, that is, a polynucleotide, are operably linked. The "operably linked" herein refers to a state in which a nucleic acid expression control sequence and a nucleic acid sequence encoding a protein or RNA of interest are functionally linked. For example, a promoter and a polynucleotide encoding a protein or RNA may be operably linked to affect the expression of the coding sequence. The operable linkage with the recombinant expression vector may be generated using genetic recombination techniques well known in the art, and site-specific DNA cutting and linking can be performed using enzymes generally known in the art.

The vectors to be used as the backbone of the recombinant expression vector herein is not particularly limited as long as it can produce the protein of the present invention. For example, it may be a plasmid DNA, phage DNA, commercially developed plasmid (pGEM^{®} T vector, pET22b, Puc18, pBAD, pIDTSAMRT-AMP, etc.), Escherichia coli-derived plasmids (pYG601BR322, pGEX-4T-1, pET, pBR325, pUC118, pUC119, etc.), Bacillus subtilis-derived plasmids (pUB110, pTP5, etc.), yeast-derived plasmids (YEp13, YEp24, YCp50, etc.), phage DNA (Charon4A, Charon21A, EMBL3, EMBL4, λgt10, AAAAAλgtH, λzap, etc.), animal viral vectors (retrovirus, adenovirus, vaccinia virus, etc.), or insect virus vector (baculovirus, etc.), but is not limited thereto.

The host cell herein includes individual cells or cell cultures that can be or have been recipients of the vector(s) for incorporation of the polypeptide insert. It includes the progeny of a single cell, which may not necessarily be completely identical (morphologically or in genomic DNA complement) to the original parent cell due to natural, accidental, or deliberate mutations. Host cells include cells that have been transfected in vivo with the polypeptide(s) of the present disclosure.

In the present invention, the host cell may include cells of mammalian, plant, insect, fungal, or cellular origin. For example, it may be bacterial cells such as Escherichia coli, Streptomyces, or Salmonella, fungal cells such as yeast cells or Pichia pastoris, insect cells such as Drosophila or Spodoptera Sf9 cells, animal cells such as Chinese hamster ovary cells (CHO), SP2/0(mouse myeloma), human lymphoblastoid, COS, NSO (mouse myeloma), 293T, Bow melanoma cells, HT-1080, baby hamster kidney cells (BHK), human embryonic kidney cells (HEK), or human retinal cells (PERC.6), or plant cells. Preferably it may be human cultured cell lines, such as a HEK cell line, but is not limited thereto, and any cell that can be used by those skilled in the art as a host cell can be used.

The composition of the present invention proliferates or activates the Pellino-1 protein, the gene encoding it, an expression vector containing the gene, or a host cell containing the expression vector, and thus inhibits the differentiation of bone marrow cells into osteoclasts when administered to a patient. Therefore, it can be applied to the prevention or treatment of bone-related diseases in which dysregulation of osteoclast differentiation is predicted.

In the pharmaceutical composition for regulating osteoclast differentiation of the present invention, contents related to the Pellino-1 protein or the gene encoding it and bone-related diseases are the same as those described in the diagnostic composition, and thus are omitted to avoid excessive complexity.

A further embodiment of the present invention is directed to a composition for inducing osteoclast differentiation that promotes the differentiation of osteoclasts.

In the present invention, the composition may comprise agents that inhibit the activity of the Pellino-1 protein or agents that reduce the expression level of the gene encoding the protein.

In the present invention, the agent that inhibits the activity of the Pellino-1 protein may be at least one selected from the group consisting of compounds, peptides, peptide mimetics, aptamers, antibodies, and natural products that specifically bind to the Pellino-1 protein, and the agent that reduces the expression level of the Pellino-1 gene may be at least one selected from the group consisting of antisense nucleotides, short interfering RNA (siRNA), short hairpin RNA, and ribozymes that complementarily binds to the Pellino-1 gene, preferably the mRNA of the gene. However, means of directly or indirectly acting on a target protein or the Pellino-1 gene to inhibit its activity or expression may be included without being limited thereto as long as it can be easily derived by known techniques commonly used in the art.

In the present invention, the "peptide minetics" is a peptide or non-peptide that inhibits the binding domain of Pellino-1 protein that leads the inhibition of Pellino-1 activity. Key residues of non-hydrolytic peptide analogs may be generated using β-turn dipeptide core (Nagai et al. Tetraheron Lett 26:647, 1985), keto-methylene pseudopeptides (Ewenson et al. J Med chem 29:295, 1986; and Ewenson et al. in Peptides: Structure and Function (Proceedings of the 9th American Peptide Symposium) Pierce chemiCal co. Rockland, IL, 1985), azepine (Huffman et al. in Peptides: Chemistry and Biology, G.R. Marshall ed., EScOM Publisher: Leiden, Netherlands, 1988), benzodiazepines (Freidinger et al. in Peptides: Chemistry and Biology, G.R. Marshall ed., EScOM Publisher: Leiden, Netherlands, 1988), β-aminoalcohols (Gordon et al. Biochem Biophys Res commun 126:419, 1985), or subsitutded gamma-lactam rings (Garvey et al. in Peptides: Chemistry and Biology, G.R. Marshall ed., EScOM Publisher: Leiden, Netherlands, 1988).

In the present invention, the "aptamer" is a single-stranded nucleic acid (DNA, RNA, or modified nucleic acid) that has stable tertiary structure and can bind to a target molecule with high affinity and specificity. Since the aptamer discovery technology SELEX (Systematic Evolution of Ligands by Exponential Enrichment) was first developed (Ellington, AD and Szostak, JW., Nature 346:818-822, 1990), many aptamers capable of binding to various target molecules such as low-molecular-weight organic substances, peptides, and membrane proteins have been continuously discovered. Aptamers are comparable to single antibodies due to their inherently high affinity (usually pM level) and their capability of specifically binding to target molecules. In particular, it has high potential as alternative antibodies to the extent that they are termed "chemical antibodies".

In the present invention, the "antibody" may be produced through injection of the protein or is commercially available. Furthermore, the antibodies include a polyclonal antibody, a monoclonal antibody, and a fragment capable of binding to an epitope, and the like.

Here, the polyclonal antibody may be produced by a conventional method of obtaining serum containing the antibody by injecting the protein into an animal and collecting blood from the corresponding animal. This polyclonal antibody may be purified by any method known in the art and made from any animal species host, such as goats, rabbits, sheep, monkeys, horses, pigs, cows, dogs, and the like.

In addition, the monoclonal antibody may be produced using any technique that provides the production of antibody molecules through continuous cell line culture. Such technologies include, but are not limited to, hybridoma technology, human B-cell line hybridoma technology, and EBV-hybridoma technology.

In addition, antibody fragments containing specific binding sites for the Pellino-1 protein may be produced. For example, F(ab')2 fragments may be produced by degrading an antibody molecule with pepsin, and Fab fragments may be produced by reducing disulfide bridges of the F(ab')2 fragments, without being limited thereto. Alternatively, monoclonal Fab fragments having desired specificity may be identified quickly and easily by decreasing a Fab expression library.

In the present invention, the antibody may be bound to a solid substrate to facilitate subsequent steps such as washing or separation of the complex. Examples of the solid substrate include a synthetic resin, nitrocellulose, a glass substrate, a metal substrate, glass fibers, microspheres, microbeads, and the like. In addition, examples of the synthetic resin include polyester, polyvinyl chloride, polystyrene, polypropylene, PVDF, nylon, and the like.

In the present invention, the agent for inhibiting the activity may specifically bind to the epitope represented by SEQ ID NO:1 of the Pellino-1 protein, and preferably, the pharmaceutical composition of the present invention may include antibodies specific to the Pellino-1 protein. Here, the antibody may specifically bind to the epitope represented as SEQ ID NO:1, but is not limited thereto.

In the present invention, the "antisense nucleotide" binds (hybridizes) to a complementary nucleotide sequence of DNA, immature-mRNA, or mature mRNA as defined in a Watson-click base pair, to interrupt the transmission of genetic information as a protein in DNA. The nature of antisense nucleotides specific to a target sequence makes them exceptionally versatile. Since the antisense nucleotides are long chains of monomer units, the antisense nucleotides may be easily synthesized with respect to the target RNA sequence. Many recent studies have verified the utility of antisense nucleotides as biochemical means for studying target proteins. Since there has been much progress in the fields of oligonucleotide chemistry and nucleotide synthesis having improved cell line adsorption, target binding affinity and nuclease resistance, the use of the antisense nucleotide may be considered as a novel type of inhibitor.

In the present invention, the "siRNA" and "shRNA" are nucleic acid molecules capable of mediating RNA interference or gene silencing, and can inhibit the expression of target genes, thereby providing an efficient gene knockdown or gene therapy method. The shRNA is a hairpin structure formed by binding of complementary sequences within a single-stranded oligonucleotide. In vivo, the shRNA is cleaved by diver to form small RNA fragments of 21 to 25 nucleotides in size to become double-stranded oligonucleotide siRNA that can specifically bind to mRNA with a complementary sequence and inhibit its expression. Therefore, hose skilled in the art can determine whether to use shRNA or siRNA, and if the mRNA sequences they target are the same, a similar reduction in expression can be expected. For the purpose of the present invention, the expression of Pellino-1 can be suppressed by specifically acting on the gene encoding the Pellino-1 protein to induce RNA interference (RNAi) by cutting the gene (e.g. mRNA molecule). siRNA may be synthesized chemically or enzymatically. The method for preparing siRNA is not particularly limited, and methods known in the art may be used. For example, there is a method for chemically synthesizing siRNA directly, a method for synthesizing siRNA using in vitro transcription, a method for cutting long double-stranded RNA synthesized by in vitro transcription using enzymes, expression methods via intracellular delivery of shRNA expression plasmids or viral vectors and expression methods through intracellular delivery of polymerase chain reaction (PCR)-induced siRNA expression cassettes, but are not limited thereto.

In the present invention, the "ribozyme" refer to RNA molecules with catalytic activity. Ribozymes with various activities are known, and the ribozymes of the Pellino-1 gene include those known or artificially produced, and optionally, ribozymes with target-specific RNA cleavage activity may be produced by known standard techniques.

The composition of the present invention promotes the differentiation of bone marrow cells into osteoclasts, and thus can be applied to the prevention or treatment of bone-related diseases in which dysregulation of osteoclast differentiation is predicted.

In the composition for inducing osteoclast differentiation of the present invention, contents related to the Pellino-1 protein or the gene encoding it, and bone-related diseases are the same as those described in the diagnostic composition, and thus are omitted to avoid excessive complexity.

Another further embodiment of the present invention is directed to a pharmaceutical composition for the treatment of bone-related diseases that inhibits the differentiation of osteoclasts.

The pharmaceutical composition herein may comprise at least one of those selected from the group consisting of the Pellino-1 protein, the gene that encodes the Pellino-1 protein, or the expression vector containing the gene encoding the Pellino-1 protein.

The pharmaceutical composition of the present invention proliferates or activates the Pellino-1 protein, the gene encoding it, an expression vector containing the gene, or a host cell containing the expression vector. Thus it can treat or prevent bone-related diseases because it inhibits osteoclast differentiation when administered to a patient.

The pharmaceutical composition of the present invention inhibits the differentiation of bone marrow cells into osteoclasts, and thus is functionally capable of preventing, ameliorating, or treating bone-related diseases. Bone-related diseases herein may be at least one of those selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis, and is preferably osteoporosis.

In addition, the pharmaceutical composition of the present invention regulates the differentiation of bone marrow cells into osteoclasts, and thus can be applied to the prevention or treatment of bone-related diseases in which dysregulation of osteoclast differentiation is predicted.

In the present invention, the "prevention" includes, without limitation, any action capable of using the composition herein to block symptoms caused by bone-related diseases, or to inhibit or delay the progression to bone-related diseases.

In the present invention, the "treatment" refers to a series of activities performed to alleviate and/or improve a target disease. For the purpose of the present invention, treatment includes activities that suppress or delay bone-related diseases, and may include, without limitation, all activities that improves or benefits symptoms caused by bone-related diseases.

In the pharmaceutical composition for the treatment of bone-related diseases of the present invention, contents related to the Pellino-1 protein or the gene encoding it, expression vectors including the gene encoding the Pellino-1 protein, and bone-related diseases are the same as those described in the diagnostic composition and pharmaceutical composition for regulating differentiation, and thus are omitted to avoid excessive complexity.

The pharmaceutical composition of the present invention may be in the form of capsules, tablets, granules, injections, ointments, powders or beverages, and may be intended for humans.

The pharmaceutical composition of the present invention, though not limited to these, may be formulated according to conventional methods into oral formulations such as powders, granules, capsules, tablets, aqueous suspensions, as well as into external preparations, suppositories, and sterile injection solutions. The pharmaceutical composition of the present invention may include a pharmaceutically acceptable carrier. Pharmaceutically acceptable carriers may include binders, lubricants, disintegrants, excipients, solubilizers, dispersants, stabilizers, suspending agents, pigments, flavors, and the like. In the case of injections, buffers, preservatives, analgesic agents, solubilizers, isotonic agents, and stabilizers may be missed and used, and in the case of local administration, bases, excipients, lubricants, preservatives, and the like may be used. The pharmaceutical composition of the present invention may be, as mentioned above, prepared in various ways by mixing with pharmaceutically acceptable carriers. For example, for oral administrations it can be prepared in the form of tablets, troches, capsules, elixirs, suspensions, syrups, wafers, etc., and for injections it can be prepared in unit dosage ampoules or multidosage forms. In addition, it may be formulated into solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative or the like.

The routes of administration of the pharmaceutical composition of the present invention include, but are not limited to, oral, intravenous, intramuscular, intraarterial, intramedullary, intrathecal, intracardiac, transdermal, subcutaneous, intraperitoneal, intranasal, enteral, topical, sublingual or rectal. Oral or parenteral administration is preferred.

The "parenteral" herein includes subcutaneous, intradermal, intravenous, intramuscular, intraarticular, intracapsular, intrasternal, intrathecal, intralesional and intracranial injection or infusion techniques. Preferably, the pharmaceutical composition of the present invention may also be administered in the form of a suppository for rectal administration, but is not limited thereto.

The pharmaceutical composition of the present invention may vary depending on various factors including the activity of the specific compound used, age, body weight, general health, sex, diet, administration time, route of administration, excretion rate, drug combination and severity of the specific disease to be prevented or treated. The dosage varies depending on the patient's condition, weight, disease severity, drug form, administration route and period but can appropriately be selected by a person skilled in the art. The pharmaceutical composition of the present invention can be administered at 0.0001 to 50mg/kg or 0.001 to 50mg/kg per day. The pharmaceutical composition can be administered once a day or may be administered in several divided dosages. The aforementioned dosages are not intended to limit the scope of the invention in any way. The pharmaceutical composition according to the present invention may be formulated as pills, sugar-coated tablets, capsules, liquids, gels, syrups, slurries, or suspensions.

Still another further embodiment of the present invention is derected to a pharmaceutical composition for the treatment of bone-related diseases that induces the differentiation of osteoclasts.

The pharmaceutical composition of the present invention may comprise agents that inhibit the activity of the Pellino-1 protein or agents that reduce the expression level of the gene encoding the protein.

The pharmaceutical composition of the present invention can treat or prevent bone-related diseases by inducing osteoclast differentiation by administering agents to patients that inhibit the activity of the Pellino-1 protein or agents that reduce the expression level of the gene encoding the protein.

The pharmaceutical composition of the present invention can induce the differentiation of bone marrow cells into osteoclasts to prevent, ameliorating, or treat bone-related diseases. The bone-related diseases may comprise osteopetrosis as a bone-related disease caused by suppression of osteoclast differentiation, but is not limited thereto.

In addition, the pharmaceutical composition of the present invention regulates the differentiation of bone marrow cells into osteoclasts, and thus may be applied to the prevention or treatment of bone-related diseases in which dysregulation of osteoclast differentiation is predicted.

In the pharmaceutical composition for the treatment of bone-related diseases of the present invention, contents related to agents that inhibit the activity of the Pellino-1 protein, agents that reduce the expression level of the gene encoding the protein, and bone-related diseases are the same as those described in the diagnostic composition and the pharmaceutical composition for regulating differentiation, and thus are omitted to avoid excessive complexity.

Yet another further embodiment of the present invention is directed to a method for treating bone-related disease, comprising a step of administering, to a subject in need thereof, an effective amount of composition containing at least one selected from the group consisting of the Pellino-1 protein (protein pellino homolog 1); the gene encoding the Pellino-1 protein; and the expression vector containing the gene encoding the Pellino-1 protein.

Still yet another further embodiment of the present invention is directed to a method for treating bone-related disease, comprising a step of administering, to a subject in need thereof, an effective amount of an agent for reducing the expression level of the Pellino-1 protein (protein pellino homolog 1) or the gene encoding the protein.

In the present invention, the term "administering" means providing a certain composition of the present invention to a subject by any suitable method.

In the present invention, the "subject" in need of administration may include both mammals and non-mammals. Here, examples of the mammals include, but are not limited to, humans, nonhuman primates such as chimpanzees, other apes or monkey species; livestock animals such as cattle, horses, sheep, goats, or pigs; domestic animals such as rabbits, dogs or cats; laboratory animals such as rodents, for example, rats, mice or guinea pigs. In addition, in the present invention, examples of the non-mammals include, but are not limited to, birds or fish.

In the present invention, the formulation of the composition that is administered as described above is not particularly limited. The composition may be administered as solid form preparations, liquid form preparations, or aerosol preparations for inhalation. Specifically, the composition may be administered as solid form preparations which are intended to be converted into liquid form preparations for oral or parenteral administration shortly before use. For example, the composition may be formulated and administered as oral preparations such as powders, granules, capsules, tablets or aqueous suspensions, as well as external preparations, suppositories, or sterile injection solutions, without being limited thereto.

In addition, in the present invention, a pharmaceutically acceptable carrier may be additionally administered together with the composition of the present invention. Pharmaceutically acceptable carriers that may be used in the present invention include binders, lubricants, disintegrants, excipients, solubilizers, dispersing agents, stabilizers, suspending agents, colorants, fragrances and the like, which may be used for oral administration; buffers, preservatives, pain-relieving agents, solubilizers, isotonic agents, stabilizers and the like, which may be used for injection; and bases, excipients, lubricants, preservatives and the like, which may be used for local administration. The composition of the present invention may be formulated in various ways by mixing it with the pharmaceutically acceptable carrier as described above. For example, for oral administration, the composition of the present invention may be formulated as tablets, troches, capsules, elixirs, suspensions, syrups, wafers or the like, and for injection, may be formulated as unit dose ampoules or multi-dose vials. In addition, the composition of the present invention may be formulated as solutions, suspensions, tablets, capsules, sustained-release preparations, or the like.

Meanwhile, examples of carriers, excipients and diluents suitable for formulation include lactose, dextrose, sucrose, sorbitol, mannitol, xylitol, erythritol, maltitol, starch, gum acacia, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate, and mineral oil. In addition, the pharmaceutical composition of the present invention may further contain a filler, an anticoagulant, a lubricant, a wetting agent, a fragrance, an emulsifier, a preservative, and the like.

The routes of administration of the composition according to the present invention include, but are not limited to, oral, intravenous, intramuscular, intraarterial, intramedullary, intradural, intracardiac, transdermal, subcutaneous, intraperitoneal, intranasal, gastrointestinal, topical, sublingual and intrarectal routes. Oral or parenteral administration is preferred.

In the present invention, the term "parenteral" is meant to include subcutaneous, transdermal, intravenous, intramuscular, intra-articular, intra-synovial, intrasternal, intradural, intra-lesional and intra-cranial injection or infusion techniques. Preferably, the pharmaceutical composition of the present invention may also be formulated as suppositories for intrarectal administration, without being limited thereto.

In the present invention, the "pharmaceutically effective amount" refers to a sufficient amount of an agent to provide the desired biological result. That result may be reduction and/or alleviation of the signs, symptoms, or causes of a disease, or any other desired alteration of a biological system. For example, an "effective amount" for therapeutic uses is the amount of the composition disclosed herein required to provide a clinically significant reduction in the disease. An appropriate effective amount in any individual case may be determined by one of ordinary skill in the art using routine experimentation. Thus, the expression "effective amount" generally refers to an amount in which the active substance has a therapeutic effect. In the case of the present invention, the active substance is an agent for regulating differentiation of bone marrow cells into osteoclasts, and at the same time, an agent for preventing, ameliorating or treating bone-related disease.

The composition of the present invention may vary depending on various factors, including the activity of the active substance used, the patient's age, body weight, general health, sex and diet, the period of administration, the route of administration, excretion rate, the drug content, and the severity of a specific disease to be prevented or treated. The dose of the active substance may be suitably selected by a person skilled in the art depending on the patient's condition and body weight, the severity of the disease, the form of drug, and the route and period of administration, and may be 0.0001 to 100 mg/kg/day or 0.001 to 100 mg/kg/day. The composition may be administered once or several times a day. The dose is not intended to limit the scope of the present invention in any way. The composition according to the present invention may be formulated as pills, sugar-coated tablets, capsules, liquids, gels, syrups, slurries, or suspensions.

Still yet another further embodiment of the present invention is directed to an agent for use in the treatment of bone-related disease, comprising at least one selected from the group consisting of the Pellino-1 protein (protein pellino homolog 1); the gene encoding the Pellino-1 protein; and the expression vector containing the gene encoding the Pellino-1 protein.

Still further embodiment of the present invention is directed to an agent for use in the treatment of bone-related disease, reducing the expression level of the Pellino-1 protein (protein pellino homolog 1) or the gene encoding the protein.

In the agent according to the present invention, contents related to the Pellino-1 protein, the gene encoding the protein, and bone-related diseases are the same as described above, and thus are omitted to avoid excessive complexity.

### [Effects of the Invention]

In the present invention, bone homeostasis can be effectively maintained by regulating bone metabolism, particularly the differentiation of bone marrow cells into osteoclasts, and thus it is very effective in preventing or treating bone-related diseases.

### [Brief Description of Drawings]

Figure 1 shows the results confirming the mRNA expression levels of Pellino-1, Pellino-2, and Pellino-3 during the osteoclast differentiation period of mice bone marrow derived macrophage (mBMM) according to an embodiment of the present invention.
Figure 2A and 2B shows changes in the NFA Tc1 promoter activity when overexpressing or knocking down Pellino-1 (protein pellino homolog 1) in HEK293T in vitro according to an embodiment of the present invention.
Figure 2C shows changes in the NFATc1 promoter activity when Pellino-1 is gradually inhibited in HEK293T in vitro according to an embodiment of the present invention.
Figure 3 shows a view of bone marrow derived microphage (BMM) separated from Pellino-1^{f1/f1} and Pellino-1^{f1/f1}Ctsk mice dyed with a tartrate resistant acid phosphatase (TRAP) and viewed under an electron microscope according to an embodiment of the present invention.
Figure 4 shows the tartrate resistant acid phosphatase (TRAP) activity of bone marrow derived microphage (BMM) separated from Pellino-1^{f1/f1} and Pellino-1^{f1/f1}Ctsk mice according to an embodiment of the present invention.
Figures 5A to 5C confirm changes in body weight and skeletal changes of a control group and a Pellino-1 deficient mouse according to an embodiment of the present invention.
Figure 6A shows the analysis of femurs of a control group and a Pellino-1 deficient mouse using a computed tomography (micro-CT) scan according to an embodiment of the present invention.
Figure 6B shows the trabecular bone volume ratio (BV/TV), bone mineral density (BMD), volumetric BMD of trabecular bone, bone surface ratio (BS/BV), trabecular thickness (Tb.Th), trabecular number (Tb.N) and trabecular separation (Tb.Sp) of a control group and a Pellino-1 deficient mouse according to an embodiment of the present invention.
Figure 7 shows the results confirming the expression level of CTX-1, a serum bone resorption index, of a control group and a Pellino-1 deficient mouse according to an embodiment of the present invention.
Figure 8 compares the degree of mRNA expression of NFATc1 after overexpressing Pellino-1 in raw cells in vitro according to an embodiment of the present invention.
Figure 9 shows an electron microscopy view after overexpression of Pellino-1 in raw cells in vitro for 1 hour, followed by staining with tartrate resistant acid phosphatase (TRAP) solution, according to an embodiment of the present invention.
Figure 10 shows the tartrate resistant acid phosphatase (TRAP) activity measured after overexpression of Pellino-1 in raw cells in vitro for 1 hour, followed by staining with tartrate resistant acid phosphatase (TRAP) solution, according to an embodiment of the present invention.

### [Best mode]

One embodiment of the present invention is directed to a composition for diagnosing bone-related disease, comprising an agent for measuring an expression level of a Pellino-1 protein (protein pellino homolog 1) or a gene encoding the protein.

Another embodiment of the present invention is directed to a kit for diagnosing bone-related disease, comprising the composition for diagnosing bone-related disease according to the present invention.

Still another embodiment of the present invention is directed to a method for providing information for diagnosing bone-related disease, comprising a step of measuring the expression level of the Pellino-1 protein (protein pellino homolog 1) or the gene encoding the protein in the biological sample isolated from a subject of interest.

Yet another embodiment of the present invention is directed to a device for diagnosing bone-related disease, the device comprising: (a) a measuring unit that measures the expression level of the Pellino-1 protein or the gene encoding the protein in the biological sample isolated from a subject of interest, and (b) a detection unit that outputs the onset or possibility of onset of bone-related diseases in the subject of interest from the measured expression levels by the measuring unit.

Still yet another embodiment of the present invention is directed to a composition for inhibiting osteoclast differentiation, comprising at least one selected from the group consisting of the Pellino-1 protein (protein pellino homolog 1); the gene encoding the Pellino-1 protein; and expression vector containing the gene encoding the Pellino-1 protein.

A further embodiment of the present invention is directed to a composition for inducing osteoclast differentiation, comprising an agent for reducing the expression level of the Pellino-1 protein (protein pellino homolog 1) or the gene encoding the protein.

Another further embodiment of the present invention is directed to a pharmaceutical composition for preventing or treating bone-related disease, comprising at least one selected from the group consisting of the Pellino-1 protein (protein pellino homolog 1); the gene encoding the Pellino-1 protein; and the expression vector containing the gene encoding the Pellino-1 protein.

Still another further embodiment of the present invention is directed to a pharmaceutical composition for preventing or treating bone-related disease, comprising an agent for reducing the expression level of the Pellino-1 protein (protein pellino homolog 1) or the gene encoding the protein.

Yet another further embodiment of the present invention is directed to a method for preventing or treating bone-related disease, comprising a step of administering, to a subject in need thereof, an effective amount of composition containing at least one selected from the group consisting of the Pellino-1 protein (protein pellino homolog 1); the gene encoding the Pellino-1 protein; and the expression vector containing the gene encoding the Pellino-1 protein.

Still yet another further embodiment of the present invention is directed to a method for preventing or treating bone-related disease, comprising a step of administering, to a subject in need thereof, an effective amount of an agent for reducing the expression level of the Pellino-1 protein (protein pellino homolog 1) or the gene encoding the protein.

A still further embodiment of the present invention is directed to an agent for use in the treatment of bone-related disease, comprising at least one selected from the group consisting of the Pellino-1 protein (protein pellino homolog 1); the gene encoding the Pellino-1 protein; and the expression vector containing the gene encoding the Pellino-1 protein.

Yet still further embodiment of the present invention is directed to an agent for use in the treatment of bone-related disease, reducing the expression level of the Pellino-1 protein (protein pellino homolog 1) or the gene encoding the protein.

### [Mode for Invention]

Hereinafter, the present invention will be described in detail by following the embodiments. These embodiments are only for explaining the present invention in more detail, and it will be apparent to those skilled in the art that the scope of the present invention is not limited by these embodiments according to the gist of the present invention.

### Preparation Example 1: Preparation of Mice

The inventors of the present invention were approved by the Yonsei University Health System Institutional Animal Care and Use Committee, and all experiments were performed with the approval of the Yonsei University College of Medicine Animal Experimentation Ethics Committee (Permit Number: 2013-0401, 2014-0033). The Pellino-1^{f1/f1}Ctsk mice were prepared by crossing the Pellino-1^{f1/f1} mice with the Pellino-1 flox/+, Cathepin K-Cre mice. Pellino-1 flox/+ mice were obtained from Korea and Cathepin K-Cre mice were obtained from the United States, but all mice were of the C57BL/6 strain.

### Preparation Example 2: Isolation of Mice Bone Marrow Derived Macrophage (mBMMS) From Mice

The bone marrow cells from 6-week old male Pellino-1^{fl/fl} and Pellino-1 Ctsk mice were isolated from the femur and tibia by flushing the bone marrow with 27 gauge needles and filtered through a 70µm cell strainer (Falcon, USA). Then the cells were centrifuged at 1,500 rpm for 10 minutes and resuspended in a α-MEM medium containing 10% FBS and 50 unit/ml of 1% penicillin. The cells were seeded in a 75T flask culture dish, and after 72 hours, the supernatant was obtained and re-inoculated into a new 10cm² culture dish. The supernatant was collected again and centrifuged at 1,500 rpm for 10 minutes, and the mouse bone marrow-derived macrophages (BMM) were finally obtained by culturing at 37°C, 5% CO₂ in α-minimum essential medium (α-MEM) containing 10% fetal bovine serum (FBS) with 1% antibiotic antifungal solution.

### Embodiment 1: Measurement of Pellino-1 Expression Level During Osteoclastogenesis

To evaluate the role of Pellino-1 in the process of osteoclastogenesis, the inventors of the present invention isolated primary osteoclast precursor cells from ICR mice and induced osteoclast differentiation in vitro by M-CSF and RANKL. At this time, BMM was isolated using Detachin^{™} (Genlantis, San Diego, CA, USA) and 1×10⁵ cells were seeded on 12 well plates for osteoclastogenesis. BMM was carried out in a growth medium containing 10 ng/ml M-CSF and 10 ng/ml RANKL to induce osteoclastogenesis and replaced every other day.

The mBMM obtained in Preparation Example 2 cultured with 10 ng/m of M-CSF and RANKL, and harvested on a designated day (right after induction and day 1 to 3 after induction). The expression levels of osteoclast marker genes were analyzed by qRT-PCR on each designated day. Total RNA used as per the manufacturer's instructions in the AccuPrep^{®} Universal RNA Extraction Kit (Bioneer), and qRT-PCR was performed to measure mRNA expression changes between groups. The primer sets used at this time are shown in Table 1 below.

**[Table 1]**

| GENE | Primer Sequence (5' → 3') |
|---|---|
| Pellino-1 | F: TGTGGGAACGTCTTCAGTCTGC |
| | R: CAGCAAGGTTGCACCACAAAGG |
| Pellino-2 | F: GTGTGACAGGAACGAGCCATAC |
| | R: AGGACACCGTTGGTAGTGAGTC |
| Pellino-3 | F: CTGCTGGCTTTGATGCCTCTAG |
| | R: GCAGAGTCCTCGGAGAAGCCA |
| β-ACTIN | F: CTTCTACAATGAGCTGCGTG |
| | R: TCATGAGGTAGTCTGTCAGG |

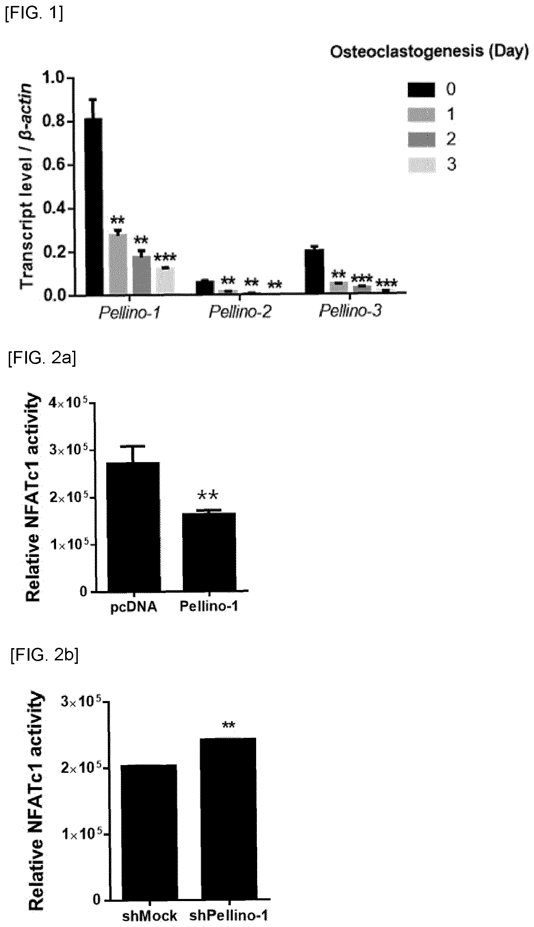

Changes in the expression of the Pellino family were confirmed during osteoclast differentiation, and Pellino-1, Pellino-2, and Pellino-3 of the Pellino family were reduced but it was confirmed that there was a significant decrease in the mRNA expression level of Pellino-1 (see Figure 1). Given such results, it can be seen that Pellino-1 of the Pellino family plays a negative role during osteoclast differentiation in mBMM.

### Embodiment 2: Verification of Osteoclast Differentiation Efficacy According to Pellino-1 Regulation

In order to verify that Pellino-1 regulation is required to activate osteoclast differentiation efficacy, we tried to confirm the correlation between regulation of Pellino-1 expression and differentiation of osteoclasts. For this, the transcriptional activity of NFATc1 (nuclear factor of activated T cell) based on regulation of Pellino-1 expression, commonly known as the most important transcription factor for osteoclast differentiation, was confirmed.

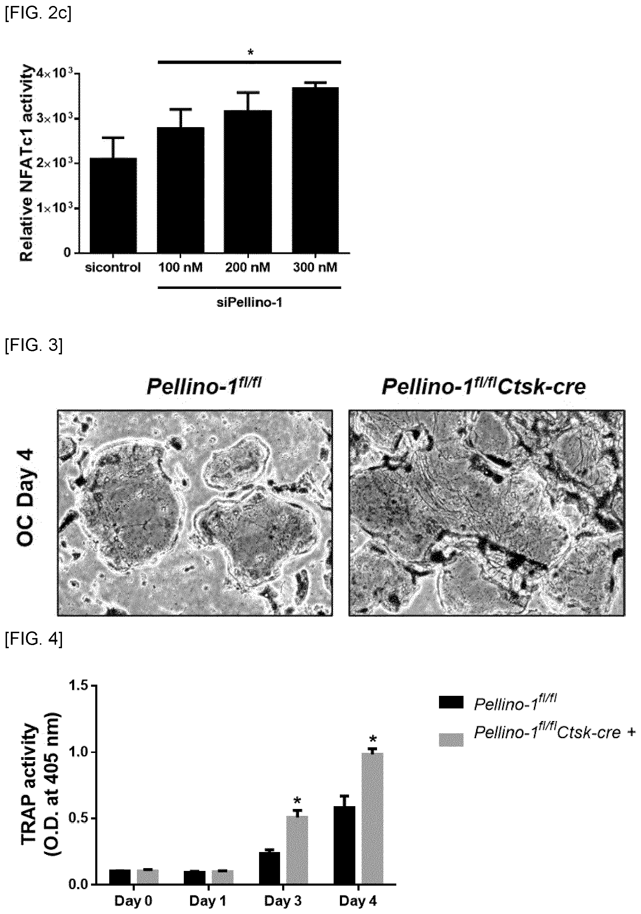

After overexpressing, knocking down, and gradually inhibiting Pellino-1 in the HEK293T cell line in vitro, the results confirming the transcriptional activity of osteoclast differentiation marker NFATc1 was shown in Figures 2A to 2C. It was confirmed that the transcriptional activity of NFATc1 decreased when Pellino-1 was overexpressed, whereas the transcriptional activity of NFA Tc1 increased when Pellino-1 was knocked down (see Figure 2A and 2B). In addition, it was confirmed that when Pellino-1 was knocked down gradually, the transcriptional activity of NFATc1 gradually increased (see Figure 2C). NFATc1 is known to play an important role in activating calcium signals in osteoclast differentiation, and when the NFATc1 transcriptional factor is activated, osteoclast differentiation is induced. As such, it can be seen that when Pellino-1 is inhibited, NFATc1 is activated and osteoclast differentiation is accelerated.

That is, the ability to activate NFATc1, a master regulator of osteoclasts, suggests that it can be used as a new target drug that can be applied to the treatment of bone and joint diseases including osteoporosis.

### Embodiment 3: Effects of Pellino-1 Deletion on Osteoclastogenesis

### 3.1 TRAP Staining and Activity Measurement

In order to see what role the deletion of Pellino-1 plays in osteoclastogenesis, an additional experiment was performed using the mice prepared in the preparation sample to confirm the effect of Pellino-1 deletion on osteoclastogenesis. Supernatant obtained from 100 µl of osteoclast culture was incubated for 1 hour at 37°C with a substrate mixture containing acetate solution (Sigma-Aldrich), 1M sodium tartrate and phosphatase substrate (Sigma-Aldrich). The reaction was stopped by adding 3 N HCl, and TRAP activity was measured at an absorbance wavelength of 405nm. At this time, TRAP staining was performed using Acid Phosphatase and Leukocyte kit (Sigma-Aldrich) according to the manufacturer's instructions, and the cells were fixed with a fixative and stained for 1 hour at 37°C.

More specifically, BMMs isolated from Pellino-1^{f1/f1} mice and Pellino-1 Ctsk mice were treated with 10 ng/ml M-CSF and 10 ng/ml RANKL to induce osteoclast differentiation, and as shown in the TRAP staining results in Figure 3, it was confirmed that osteoclast differentiation increased in Pellino-1 Ctsk BMM in comparison to Pellino-1^{f1/f1} BMM. In addition, it was confirmed that TRAP activity was significantly increased in Pellino-1 Ctsk BMM compared to in Pellino-1^{f1/f1} BMM (see Figure 4).

### 3.2 Confirmation of in vivo Function of Pellino-1 (Confirmation of Body Weight and Skeletal Changes)

In order to confirm the in vivo function of Pellino-1 in osteoclasts, experiments were performed with the mice from Preparation Example 2 from birth to 12 weeks. Femur specimen were fixed in 70% ethanol for 24 hours at room temperature for micro-CT (µCT) analysis, and the fixed sample were analyzed using high-resolution µCT (Skyscan-1173, Skyscan, Kontich, Belgium). µCT image reconstruction and analysis were performed using the reconstruction software Nrecon (v1.7.0.4, Bruker-CT), cross-sectional images were performed with DataViewer (v1.5.1.2, Bruker-CT), and measurements were performed with SkyScan1173 control software (v1.6, Bruker-CT).

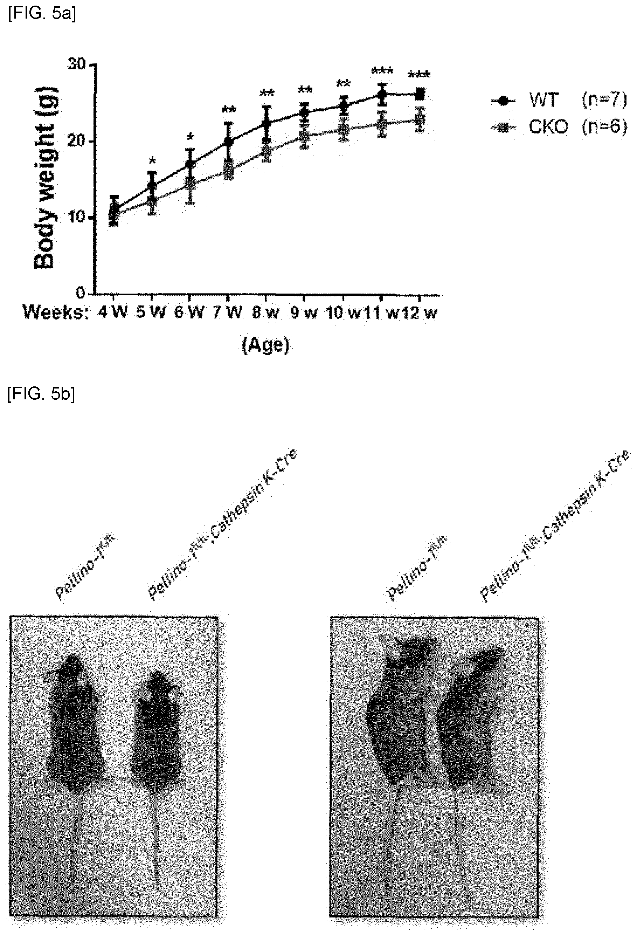

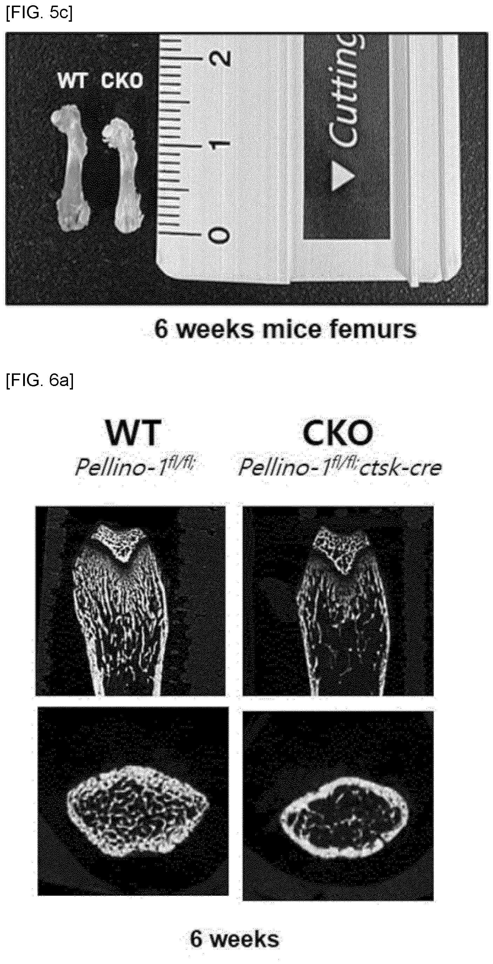

Pellino-1 Ctsk in the femur of 6-week-old mice had no effect on viability and fertility. From the 5th week, the body weight of Pellino-1Ctsk was lower than that of Pellino-1^{f1/f1}, and at week 6, it was confirmed that the size of Pellino-1 Ctsk was smaller than that of Pellino-1^{f1/f1}. In addition, it was confirmed that the size of the femur was smaller in Pellino-1 Ctsk compared to Pellino-1^{f1/f1} mice (see Figures 5A to 5C). However, there was no difference in neonatal mice, suggesting that regulation of osteoclast activity by Pellino-1 was performed during postnatal development.

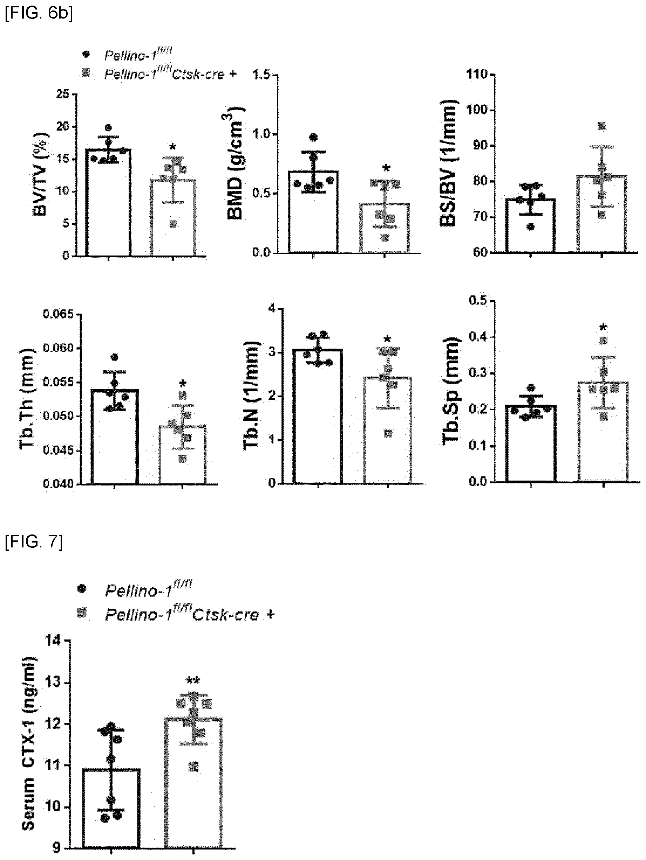

The trabecular bone volume ratio (BV/TV), bone mineral density (BMD), volumetric BMD of trabecular bone, bone surface ratio (BS/BV), trabecular thickness (Tb.Th), trabecular number (Tb.N) and trabecular separation (Tb.Sp) of 6-week-old Pellino-1 Ctsk mice were measured. The femur showed a significant decrease in bone volume compared to the control group, and in bone, Pellino-1 Ctsk mice were characterized by significantly reduced trabecular bone volume ration, bone density, trabecular thickness, trabecular number, and trabecular separation compared to the Pellino-1^{f1/f1} mice (see Figure 6B). These results suggest that Pellino-1 deletion in osteoclasts induces excessive bone resorption.

### 3.3 Measurement of The Level of Bone Resorption

To confirm the effect of Pellino-1 deletion on osteoclast development, serum levels of CTX (collage type I) c-terminal telopeptides, a bone resorption marker, were measured in the Pellino-1 Ctsk group. Immediately after sacrifice of the mice, blood was obtained from 6-week-old male Pellino-1^{f1/f1} and Pellino-1 Ctsk mice, and the serum was separated using a serum separator tube, and serum levels of CTX (IDS) were measured according to the serum bone resorption index using each ELISA kit according to the manufacturer's instructions.

The Pellino-1 knockout was found to increase bone resorption (see Figure 7). Increased serum levels of CTX-1, a diagnostic marker for bone resorption, were increased in the Pellino-1 deletion mouse group, confirming increased bone resorption, suggesting that the downregulation of Pellino-1 is an important process for osteoclast differentiation.

### 3.4 Statistical Analysis

All statistical analyses in the present invention were performed by analysis of variance (ANOVA) or Student's t-test using GraphPad Prism6 software, and each data was calculated as mean ± standard deviation. A p-value of less than 0.05 was considered statistically significant.

### Embodiment 4: Effect of Pellino-1 Overexpression On Osteoclastogenesis

### 4.1 Measurement of NFA Tc1 mRNA Expression Level

In order to see what role Pellino-1 overexpression plays in the process of osteoclastogenesis, an additional in vitro experiment was performed to confirm the effect of Pellino-1 overexpression on osteoclastogenesis. Specifically, after overexpressing Pellino-1 in RAW 264.7 cells using a lentiviral vector, differentiation of osteoclasts was induced for 4 days. On day 4 of differentiation, RT-PCR was performed to compare and analyze the mRNA expression level of NFATc1, known as the most important transcription factor for osteoclast differentiation.

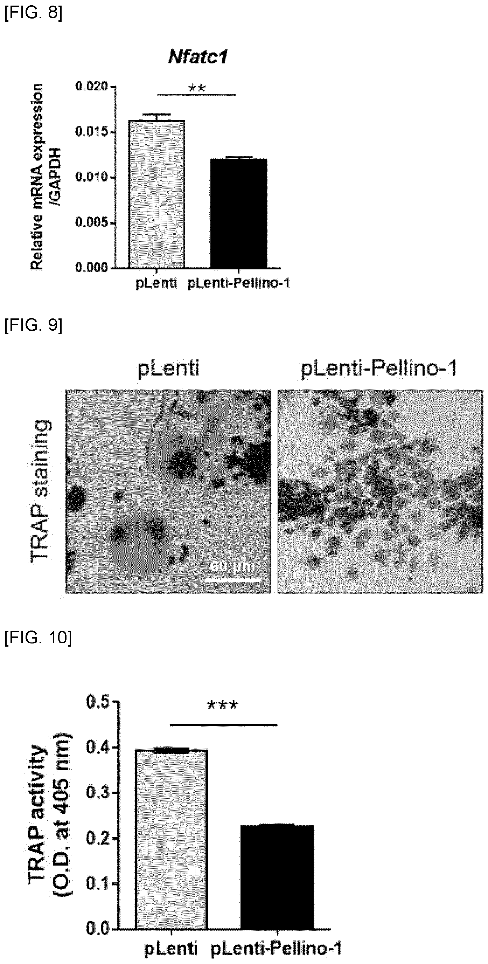

The results of the experiment confirmed that the mRNA expression level of NFATc1 was inhibited in the Pellino-1 overexpressed group compared to the control group (see Figure 8). From this, it can be seen that the differentiation of osteoclasts will be suppressed through the decreased expression of the NFA Tc1 transcriptional factor in the Pellino-1 overexpressed group.

### 4.2 TRAP Staining and Activity Measurement

In order to confirm the osteoclastogenesis inhibitory effect upon Pellino-1 overexpression, an additional experiment was performed according to the method in Embodiment 3 using the RAW 264.7 cells prepared in 4.1 above. Specifically, the supernatant obtained from 100 µl of cell culture was incubated for 1 hour at 37°C with a substrate mixture containing acetate solution (Sigma-Aldrich), 1M sodium tartrate and phosphatase substrate (Sigma-Aldrich). The reaction was stopped by adding 3 N HCl, and TRAP activity was measured at an absorbance wavelength of 405nm. At this time, TRAP staining was performed using Acid Phosphatase and Leukocyte kit (Sigma-Aldrich) according to the manufacturer's instructions, and the cells were fixed with a fixative and stained for 1 hour at 37°C.

The results of the experiment confirmed that as for osteoclast differentiation, the proportion of TRAP (+) cells decreased in the Pellino-1 overexpressed group as shown in the TRAP staining results of Figure 9. As shown in Figure 10, it was confirmed that the differentiation potential was inhibited through the decrease in TRAP activity. By confirming the effect of Pellino-1 overexpression, it was confirmed again that it was a negative regulator of osteoclast differentiation,

Taken together, it can be confirmed that the deletion of Pellino-1 promotes differentiation of osteoclasts, and overexpression of Pellino-1 inhibits differentiation of osteoclasts. This suggests that using Pellino-1 as a positive/negative regulator that regulates the differentiation of osteoclasts can treat bone-related diseases caused by dysregulation of osteoclast differentiation. Therefore, Pellino-1 is expected to be a new therapeutic target for bone-related diseases beyond bone-related diagnosis.

Having described specific parts of the present invention in detail above, it is clear to those skilled in the art that these specific techniques are only preferred embodiments, and that the scope of the present invention is not limited thereto. Accordingly, the substantial scope of the present invention will be defined by the appended claims and equivalents thereof.

### [Industrial Applicability]

A composition according to the prevent invention can maintain bone homeostasis by regulating bone metabolism, particularly the differentiation of bone marrow cells into osteoclasts, and can also be used very effectively for preventing or treating bone-related diseases.

## Claims

1. A composition for diagnosing bone-related disease, comprising an agent for measuring an expression level of a Pellino-1 protein (protein pellino homolog 1) or a gene encoding the protein.

2. The composition of claim 1,
wherein the Pellino-1 protein has the amino acid sequence represented by SEQ ID NO: 1.

3. The composition of claim 1,
wherein the Pellino-1 gene has the nucleic acid sequence represented by SEQ ID NO:2.

4. The composition of claim 1,
wherein the agent for measuring the expression level of the protein comprises at least one selected from the group consisting of an antibody, an oligopeptide, a ligand, a peptide nucleic acid (PNA), and an aptamer, which bind specifically to the protein.

5. The composition of claim 1,
wherein the agent for measuring the expression level of the gene comprises at least one selected from the group consisting of a primer, a probe, and an antisense nucleotide, which bind specifically to the gene.

6. The composition of claim 1,
wherein the bone-related disease comprises at least one selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis.

7. The composition of claim 1,
wherein the bone-related disease is caused by abnormal differentiation of bone marrow cells into osteoclasts.

8. A kit for diagnosing bone-related disease, comprising the composition of any one of claims 1 to 7.

9. The kit of claim 8,
wherein the kit comprises at least one selected from the group consisting of RT-PCR kit, DNA ChIP kit, ELISA kit, protein ChIP kit, rapid kit, or multiple reaction monitoring (MRM) kit.

10. A method for providing information for diagnosing bone-related disease, comprising a step of measuring the expression level of the Pellino-1 protein (protein pellino homolog 1) or the gene encoding the protein in the biological sample isolated from a subject of interest.

11. The method of claim 10,
wherein it is predicted that, when the measured expression level of the Pellino-1 protein or the gene encoding the protein in the biological sample is higher than a control, the subject of interest has bone-related disease due to inhibition of osteoclast differentiation.

12. The method of claim 10,
wherein it is predicted that, when the measured expression level of the Pellino-1 protein or the gene encoding the protein in the biological sample is lower than a control, the subject of interest has bone-related disease due to hyperdifferentiation of osteoclast.

13. A device for diagnosing bone-related disease, the device comprising:
(a) a measuring unit that measures the expression level of the Pellino-1 protein or the gene encoding the protein in the biological sample isolated from a subject of interest, and
(b) a detection unit that outputs the onset or possibility of onset of bone-related diseases in the subject of interest from the measured expression levels by the measuring unit.

14. A composition for inhibiting osteoclast differentiation, comprising at least one selected from the group consisting of the Pellino-1 protein (protein pellino homolog 1); the gene encoding the Pellino-1 protein; and expression vector containing the gene encoding the Pellino-1 protein.

15. A composition for inducing osteoclast differentiation, comprising an agent for reducing the expression level of the Pellino-1 protein (protein pellino homolog 1) or the gene encoding the protein.

16. The composition of claim 15,
wherein the agent for reducing the expression levels is a protein activity inhibitor or a gene expression inhibitor.

17. The composition of claim 16,
wherein the protein activity inhibitor is any one or more selected from the group consisting of compounds, peptides, peptide mimetics, aptamers, antibodies, and natural products, which bind specifically to the Pellino-1 protein.

18. The composition of claim 16,
wherein the gene expression inhibitor is any one or more selected from the group consisting of an antisense nucleotide, a short interfering RNA (siRNA), a short hairpin RNA, and ribozymes, which bind complementarily to the Pellino-1 gene.

19. A pharmaceutical composition for treating bone-related disease, comprising at least one selected from the group consisting of the Pellino-1 protein (protein pellino homolog 1); the gene encoding the Pellino-1 protein; and the expression vector containing the gene encoding the Pellino-1 protein.

20. The composition of claim 19,
wherein the bone-related disease comprises at least one selected from the group consisting of osteoporosis, osteomalacia, osteopenia, osteopetrosis, bone atrophy, fibrous dysplasia, Paget's disease, hypercalcemia, giant cell tumor, neoplastic destruction, cancer-related bone resorption disease, fracture, osteolysis, osteoarthritis, and rheumatoid arthritis.

21. The composition of claim 19,
wherein the bone-related disease is caused by hyperdifferentiation of bone marrow cells into osteoclasts.

22. A pharmaceutical composition for treating bone-related disease, comprising an agent for reducing the expression level of the Pellino-1 protein (protein pellino homolog 1) or the gene encoding the protein.

23. The composition of claim 22,
wherein the bone-related disease is osteopetrosis.

24. The composition of claim 22,
wherein the bone-related disease is caused by inhibition of differentiation of bone marrow cells into osteoclasts.

25. A method for preventing or treating bone-related disease, comprising a step of administering, to a subject in need thereof, an effective amount of composition containing at least one selected from the group consisting of the Pellino-1 protein (protein pellino homolog 1); the gene encoding the Pellino-1 protein; and the expression vector containing the gene encoding the Pellino-1 protein.

26. A method for preventing or treating bone-related disease, comprising a step of administering, to a subject in need thereof, an effective amount of an agent for reducing the expression level of the Pellino-1 protein (protein pellino homolog 1) or the gene encoding the protein.

27. An agent for use in the treatment of bone-related disease, comprising at least one selected from the group consisting of the Pellino-1 protein (protein pellino homolog 1); the gene encoding the Pellino-1 protein; and the expression vector containing the gene encoding the Pellino-1 protein.

28. An agent for use in the treatment of bone-related disease, reducing the expression level of the Pellino-1 protein (protein pellino homolog 1) or the gene encoding the protein.
